# EUROPEAN PATENT APPLICATION

(11) **EP 4 783 188 A1**
(43) Date of publication of application: **29.07.2026**
(21) Application number: 25153488.9
(22) Date of filing: 23.01.2025
(51) Int. Cl.: G16H 40/20, G06Q 10/1093, G16H 40/63, G16H 50/30

(54) **DYNAMIC MULTI-DIMENSIONAL HEALTH MONITORING SYSTEM FOR PERSONALIZED DIABETES MANAGEMENT**

(71) Applicant: OneTwo Analytics AB, 112 27 Stockholm (SE)
(72) Inventor: Dawnbringer, Jeanie, 112 27 Stockholm (SE); Carlsson, Per-Ola, 112 27 Stockholm (SE); Cederblad, Lars, 112 27 Stockholm (SE); Espes, Daniel, 112 27 Stockholm (SE); Hugert, Fabian, 112 27 Stockholm (SE)
(74) Representative: Høiberg P/S

(57) **Abstract**

The invention relates to a system for managing diabetes care for a patient. The system includes a data acquisition module configured to collect diabetes health data, such as glucose monitoring data, electronic health records, patient-reported diabetes data, and fitness data. A score-target interval mapping module calculates health dimension scores for glucose control, cardiovascular health, and diabetes resilience and maps each score to a corresponding health target interval using dimension-specific mapping functions. An appointment management module calculates target dates based on the elapsed time since the most recent appointment and the health target intervals. The module determines appointment parameters, including a primary target date based on the triggering health dimension with the earliest target date and assigns a healthcare provider role for the follow-up care appointment.

## Description

The present disclosure relates to a system for personalized diabetes health management, and related devices and methods.

### Background

Diabetes management is a complex and ongoing process that requires regular monitoring and adjustments to meet the unique needs of each patient. Current methods of diabetes care rely on health metrics, such as blood glucose levels, or cardiovascular health markers, which are often collected from various sources. However, the lack of integrated, multi-dimensional systems in diabetes management often results in fragmented data that do not provide a comprehensive view of a patient's health, making it challenging to address the specific needs of each individual.

A significant limitation of conventional diabetes management systems is the use of standardized follow-up intervals, which often apply a "one-size-fits-all" approach to scheduling. This means that patients with vastly different health profiles or changing needs may receive the same standard follow-up frequency, which may not align with their actual condition.

Such uniform intervals can lead to delayed care for high-risk patients, while low-risk patients may be seen more frequently than necessary. This lack of customization in scheduling follow-ups reduces the effectiveness of diabetes care and increases the potential for preventable complications.

In addition, prior art systems typically lack the flexibility to adjust healthcare provider roles and responsibilities according to a patient's evolving condition. Different aspects of diabetes management, such as cardiovascular health and glucose control, may require different levels of expertise, yet conventional systems do not account for this variation. Instead, appointments are often managed without adapting the provider's role to meet the specific needs associated with each health metric, which can lead to suboptimal treatment plans and inefficient use of healthcare resources.

Moreover, the limited responsiveness of current systems to recent changes in a patient's health data further impacts the quality of diabetes care. As a result, critical changes in a patient's health can go unaddressed until a scheduled appointment, potentially worsening their condition. Without a means to account for these factors, existing methods in diabetes care often fall short of providing the responsive and individualized management needed to ensure optimal patient outcomes.

### Summary

The present inventors have realized that effective diabetes management requires a comprehensive approach that integrates multi-dimensional health data in order to enable personalized and responsive care.

Therefore, the present disclosure relates to a computer-implemented method for managing diabetes care for a patient, comprising:
- collecting diabetes health data of the patient, including:
   ∘ glucose monitoring data,
   ∘ electronic health records,
   ∘ patient-reported diabetes data, and
   ∘ fitness data;
- calculating a health dimension score for each of a set of health dimensions derived from the collected diabetes health data, wherein the health dimensions include glucose control, cardiovascular health, and diabetes resilience;
- mapping each health dimension to a health target interval for a follow-up care appointment, based on each health dimension score and a mapping function specific to each health dimension, the health target intervals comprising a glucose control target interval, a cardiovascular health target interval, and a diabetes resilience target interval;
- calculating target dates for each health dimension based on the elapsed time since the most recent appointment for that health dimension and its corresponding health target interval;
- determining a triggering health dimension, which is the health dimension with the earliest target date among the diabetes-specific health dimensions;
- determining appointment parameters for the follow-up care appointment, wherein the appointment parameters include:
   ∘ a primary target date for the follow-up care appointment, determined based on the triggering health dimension, and
   ∘ a healthcare provider role for the follow-up care appointment, determined based on the triggering health dimension;
providing a notification of the determined follow-up care appointment and the appointment parameters.

In a further aspect, the present disclosure relates to a system for managing diabetes care for a patient, the system comprising:
- a data acquisition module configured to collect diabetes health data of the patient, including glucose monitoring data, electronic health records, patient-reported diabetes data, and fitness data;
- a score-target interval mapping module configured to:
   ∘ calculate a health dimension score for each of a set of health dimensions derived from the collected diabetes health data, wherein the health dimensions include glucose control, cardiovascular health, and diabetes resilience; and
   ∘ map each health dimension to a health target interval for a follow-up care appointment, based on each health dimension score and a mapping function specific to each health dimension, the health target intervals comprising a glucose control target interval, a cardiovascular health target interval, and a diabetes resilience target interval;
- an appointment management module configured to:
   ∘ calculate target dates for each health dimension based on the elapsed time since the most recent appointment for that health dimension and its corresponding health target interval;
   ∘ determine a triggering health dimension which is the health dimension with the earliest target date;
   ∘ determine appointment parameters for the follow-up care appointment, wherein the appointment parameters includes:
      ∘ a primary target date for the follow-up care appointment, determined based on the triggering health dimension, among the diabetes-specific health dimensions; and
      ∘ a healthcare provider role for the follow-up care appointment, determined based on the triggering health dimension.

In yet another aspect, the present disclosure relates to a computer program product comprising instructions which, when executed by a processor, cause a computer to carry out the method as disclosed herein.

In a further aspect, the present disclosure relates to a device for managing diabetes care for a patient, comprising:
- a data acquisition module configured to collect diabetes health data of the patient, including glucose monitoring data, electronic health records, patient-reported diabetes data, and fitness data;
- a processing unit configured to:
   ∘ calculate a health dimension score for each of a set of health dimensions derived from the collected diabetes health data, wherein the health dimensions include glucose control, cardiovascular health, and diabetes resilience;
   ∘ map each health dimension to a health target interval for a follow-up care appointment, based on each health dimension score and a mapping function specific to each health dimension, the health target intervals comprising a glucose control target interval, a cardiovascular health target interval, and a diabetes resilience target interval;
   ∘ calculate target dates for each health dimension based on the elapsed time since the most recent appointment for that health dimension and its corresponding health target interval;
   ∘ determine appointment parameters for the follow-up care appointment, including a primary target date for the follow-up care appointment, determined based on a triggering health dimension which has the earliest target date among the diabetes-specific health dimensions, and a healthcare provider role for the follow-up care appointment, determined based on the triggering health dimension.

The disclosed method, system, computer program product, and device enable an individualized approach to diabetes management by leveraging real-time and patient-specific measurement data actively collected from various sources, such as continuous glucose monitors (CGMs) and fitness or activity trackers. These measurement devices provide crucial data inputs, including glucose monitoring, physical activity, and other health metrics, which form the basis for dynamic calculations of health dimension scores across glucose control, cardiovascular health, and diabetes resilience. Each health dimension score is mapped to a specific target interval, allowing the system to respond effectively to changes in the patient's health status and ensuring that high-priority conditions receive timely follow-up appointments.

The appointment management module further enhances the adaptability of the disclosed solution by calculating specific target dates for each health dimension, taking into account the time elapsed since the last appointment and the assigned target interval. The module identifies a triggering health dimension-typically the dimension with the earliest target date-to set a primary target date for the follow-up care appointment. This triggering mechanism enables the system to prioritize care based on the most urgent health needs, ensuring that the selected healthcare provider role is best suited to address the identified priority.

This tailored approach to diabetes care management addresses gaps in current methods by allowing for flexible, responsive scheduling and provider role assignment. The disclosed technology's capacity to dynamically adjust to real-time health changes offers significant advantages in preventing delays in treatment, improving patient outcomes, and supporting a more resource-efficient model of healthcare delivery.

By actively collecting patient data from measurement devices and integrating these data with electronic health records and patient-reported inputs, the present disclosure provides a comprehensive and responsive solution for individualized diabetes care that is adaptable to the specific needs and evolving conditions of each patient.

### Description of the drawings

In the following embodiment and examples will be described in greater detail with reference to the accompanying drawings:
Fig. 1 shows a flowchart outlining an example of a method for managing diabetes care according to an embodiment of the present disclosure,
Fig. 2 illustrates an embodiment of a mapping function for determining the cardiovascular health (CVH) target interval based on the cardiovascular health score as disclosed herein.
Fig. 3 illustrates an embodiment of a mapping function for determining the glucose control (GLC) target interval based on the glucose control score as disclosed herein.
Fig. 4 illustrates an embodiment of a mapping function for determining the diabetes resilience (DR) target interval based on the diabetes resilience score as disclosed herein.
Fig. 5 illustrates a diagram showing the linkage between health dimensions and healthcare provider roles, including primary and secondary responsibilities, as disclosed herein.

### Detailed description

### Definitions

The term diabetes health data, as used herein, refers to information pertinent to the management of diabetes, encompassing a range of clinical and patient-reported metrics collected from various sources. It provides a comprehensive overview of the patient's condition to inform and optimize care.

The term glucose monitoring data, as used herein, refers to information related to the measurement of blood glucose levels over time. This data may be obtained from continuous glucose monitoring (CGM) devices, self-monitored blood glucose (SMBG) readings logged by the patient, or laboratory measurements such as HbA1c levels, which reflect average blood glucose control over several weeks.

The term electronic health records (EHRs), as used herein, refers to structured clinical information stored digitally, including laboratory test results, blood pressure readings, medical history, prescribed medications, and demographic details such as age, sex, and relevant social or lifestyle factors.

The term patient-reported diabetes data, as used herein, refers to information provided directly by the patient concerning their health and lifestyle. Examples include responses to diabetes-specific questionnaires, daily glucose logs, blood pressure measurements, descriptive data such as weight, and other lifestyle-related factors relevant to diabetes care.

The term fitness data, as used herein, refers to metrics related to the patient's physical activity and overall fitness, which may influence diabetes management. Examples include step counts, caloric expenditure, heart rate data, exercise duration, and sleep patterns, often collected via wearable devices or fitness tracking systems.

In a first aspect, the present disclosure relates to a computer-implemented method for managing diabetes care for a patient.

The method can comprise collecting diabetes health data of the patient. In one example, the diabetes health data comprises one or more of glucose monitoring data, electronic health records, patient-reported diabetes data, and fitness data.

The method may further comprise a step of calculating a health dimension score for each of a set of health dimensions derived from the collected diabetes health data. Said health dimensions may for example include glucose control, cardiovascular health, and diabetes resilience.

The method may further include a step of calculating target dates for each health dimension. The target dates may for example be calculated based on the elapsed time since the most recent appointment for that health dimension and its corresponding health target interval.

The presently disclosed method may further comprise a step of determining a triggering health dimension. Said triggering health dimension may for example be the health dimension with the earliest target date among the diabetes-specific health dimensions.

The presently disclosed method may further comprise a step of determining appointment parameters for the follow-up care appointment. The appointment parameters may for example comprise a primary target date for the follow-up care appointment, and/or a healthcare provider role for the follow-up care appointment. The primary target date for the follow-up care appointment may be determined based on the triggering health dimension. The healthcare provider role for the follow-up care appointment, can be determined based on the triggering health dimension.

The method may further comprise a step of providing a notification of the determined follow-up care appointment and the appointment parameters.

The presently disclosed method provides a technical solution to the shortcomings of prior art in managing diabetes care by integrating multi-dimensional health data, dynamically mapping health dimensions to follow-up intervals, and tailoring appointment scheduling to the specific needs of patients. This approach overcomes the rigid, one-size-fits-all scheduling of traditional methods and the inefficiencies of fragmented data management.

By collecting diabetes health data from diverse sources, including glucose monitoring data, electronic health records, patient-reported diabetes data, and fitness data, the disclosed method ensures a comprehensive view of the patient's health. This eliminates the data fragmentation seen in prior methods, where isolated metrics failed to provide a holistic understanding of the patient's condition. The ability to actively collect real-time data, such as from continuous glucose monitoring devices or activity trackers, further enhances responsiveness to dynamic changes in patient health.

The method calculates health dimension scores for a set of health dimensions-glucose control, cardiovascular health, and diabetes resilience-based on the collected diabetes health data. These scores reflect the current state of each health dimension and allow for an individualized assessment. Mapping each health dimension score to a health target interval through a mapping function specific to each dimension introduces a level of customization absent in prior art. This mapping ensures that patients at higher risk receive follow-up care sooner, while those with stable conditions avoid unnecessary appointments.

Determining a triggering health dimension, which corresponds to the earliest target date among the diabetes-specific health dimensions, allows the method to prioritize the most urgent aspect of the patient's health. The method then determines appointment parameters, including a primary target date and a healthcare provider role, ensuring that appointments are aligned with the patient's immediate needs and the expertise required for care. This flexibility addresses the inefficiencies of prior methods that failed to adapt provider roles based on evolving patient conditions.

Providing a notification of the determined follow-up care appointment and its parameters ensures that both patients and healthcare providers are informed promptly, fostering timely interventions and improving adherence to care plans. This feature also supports coordination among healthcare providers, optimizing the use of clinic resources.

The method may involve collecting diabetes health data from various sources relevant to the patient's condition. This data may include glucose monitoring data, electronic health records (EHRs), patient-reported diabetes information, and fitness data. By incorporating diverse sources of data, the method achieves a comprehensive understanding of the patient's health profile, enabling tailored care based on up-to-date health metrics.

For example, the collected diabetes health data can comprise real-time measurement data from one or more patient monitoring devices, such as a continuous glucose monitoring (CGM) device, activity tracker, and self-monitoring blood glucose device. These devices provide accurate, real-time data that reflects the patient's current health status, allowing the method to respond dynamically to any variations in the patient's condition. Active data collection from such devices ensures that the patient's care regimen can be adjusted in real-time, thus facilitating a responsive approach to diabetes management.

The diabetes health data collected from EHRs can include laboratory test results, chronic condition records, demographic information, medical history, prescribed medications, and/or blood pressure readings. Access to such comprehensive EHR data allows for an in-depth analysis of the patient's health status, contributing to a more holistic and accurate health dimension score. This integration supports more precise adjustments to care, considering both current and historical health indicators.

The patient-reported diabetes data can comprise responses to diabetes-specific questionnaires, blood glucose entries, blood pressure measurements, and/or other descriptive data such as weight and age. Patient-reported data provides insights into the subjective experiences and daily health metrics that may not be captured in clinical measurements. This information is useful for tracking the patient's self-reported symptoms and daily management habits, contributing valuable data for calculating health dimension scores.

The patient-reported diabetes data provides a valuable, patient-centric perspective that complements clinical records. Responses to diabetes-specific questionnaires, such as the PAID questionnaire, can quantify psychosocial factors impacting diabetes management. Blood glucose entries allow patients to contribute real-time or historical self-monitoring data, which may reflect trends not captured in clinic visits. Blood pressure measurements and descriptive data, such as weight and age, enhance the dataset's granularity, enabling a more nuanced assessment of the patient's overall condition.

This patient-driven approach fosters engagement and empowers patients to take an active role in their diabetes management. By incorporating patient-reported data, the method enhances the comprehensiveness of the health dimension scores, which, in turn, leads to more accurate and personalized follow-up care recommendations. Additionally, patient-reported data facilitates real-time insights, enabling the method to adapt to sudden changes in the patient's condition that might otherwise go unnoticed.

The patients can input data, such as the patient-reported data, via a companion application or dedicated interface, such as a smartphone app, a web portal, or a wearable device. In some embodiments, the patient-reported data may be validated against historical trends or clinical thresholds to ensure consistency. Alternative implementations may include integration with remote monitoring devices that automatically populate patient records, reducing manual input errors.

The fitness data can include physical activity metrics, sleep patterns, heart rate data, and caloric expenditure, which are typically gathered from fitness tracking devices. This information can complement the clinical and/or patient-reported data by providing context on the patient's physical activity and overall lifestyle. Understanding the patient's activity levels and sleep quality can aid in predicting fluctuations in glucose levels and other health metrics, thereby enhancing the accuracy of the health dimension scores. As such, integrating fitness data allows the method to provide more actionable recommendations for follow-up care and health improvements. For instance, a lack of activity may prompt the system to recommend lifestyle changes or escalate care urgency. This dynamic inclusion of fitness metrics ensures that diabetes care extends beyond clinical metrics, addressing the broader aspects of patient health.

In one embodiment, calculating the health dimension scores may involve applying weightings to specific data inputs based on their clinical relevance to the patient's condition. The weightings may prioritize certain health indicators over others, depending on the unique clinical profile and needs of the patient. For example, patients with a history of cardiovascular disease may have weightings applied to blood pressure and cholesterol data that are higher than weightings applied to other metrics, such as physical activity. This approach enables the system to focus on the most critical health indicators for each individual, improving the relevance and accuracy of the health dimension scores.

The weightings may be predefined according to standardized clinical guidelines, such as those issued by national or international health organizations, ensuring consistency with best practices in diabetes management. Alternatively, healthcare providers may customize the weightings to address the specific needs of their patient populations or individual cases. For instance, providers treating elderly patients may prioritize metrics such as blood pressure and mobility, while those managing younger, active patients may emphasize fitness-related metrics.

In some embodiments, the weightings may be dynamically adjusted based on changes in the patient's health data. For example, if a patient's glucose levels become unstable or if new cardiovascular risk factors are identified, the system may automatically increase the weightings for glucose monitoring data or cardiovascular health metrics. This real-time adaptability ensures that the scoring methodology remains aligned with the patient's evolving health status, enabling proactive and responsive care management.

By applying weightings to the diabetes health data, the method achieves a scoring system that is tailored, clinically meaningful, and adaptable. This approach enhances the specificity and reliability of the health dimension scores, ensuring that follow-up care recommendations are both accurate and personalized. Additionally, the flexibility of the weighted scoring methodology allows healthcare providers to align the scoring system with evolving medical practices, emerging guidelines, or individual patient needs, further improving the quality of diabetes care.The health dimension scores can be updated periodically to account for newly collected health data. This can allow the method to adapt dynamically to changes in the patient's condition. Regular score updates ensure that the patient's care plan remains relevant and responsive, avoiding outdated assessments that may lead to gaps in care. Dynamic score adjustment is advantageous for continuous care, as it aligns healthcare interventions with the patient's current health needs.

Each health dimension score can be mapped to a specific health target interval. For example by using a mapping function that assigns a variable target interval based on urgency level. For example, the mapping function may define shorter intervals for more urgent health scores, indicating the need for urgent follow-up, and longer intervals for better health scores, reflecting reduced urgency. This mapping ensures that each patient's needs are met with a frequency that aligns with the urgency of their health status.

The mapping function for each health dimension score may be configured as a piecewise function with at least three intervals, each associated with a specific subfunction, which are different and/or the same. The first interval may correspond to scores indicating the most critical health status, the second interval may address moderate or transitional states, and the third interval may reflect stable or non-critical conditions. This structure allows for differentiated responses based on specific score ranges, enabling more granular control over the intervals assigned for follow-up care. Such configurations enhance the method's adaptability, ensuring that high-risk patients receive prompt care while minimizing unnecessary visits for low-risk patients.

In one example, the piecewise function's first and third subfunctions are constant functions, establishing fixed health target intervals for each respective range, while the second subfunction has a non-zero derivative over the second interval. This can be one way to enable stable intervals for extreme health states (e.g., critically low or high scores), while introducing flexibility and fine-tuning for intermediate scores that are more likely to fluctuate. A non-zero derivative in the middle interval enables a more responsive adjustment of the target interval as the patient's score changes within that range, offering a nuanced approach to care frequency.

In cases where the one or more health dimension scores are unknown, the corresponding health target intervals are assigned a predetermined default value. For example, if glucose control data is unavailable due to e.g. device malfunction or patient non-compliance, the method may assign a conservative interval to ensure timely follow-up. This feature ensures that the method can proceed with follow-up scheduling, even when complete data is unavailable, reducing the risk of missed or delayed care due to data gaps. By implementing a default interval, the method maintains continuity in care management.

The method may include adjusting one or more health target intervals based on the presence of known complications, such as retinopathy, cardiovascular disease, or foot ulcers. Such adjustments may include shortening the interval for patients with severe or progressive conditions, ensuring that necessary care is provided promptly. This adjustment may involve shortening the health target interval for affected dimensions, thereby increasing the frequency of monitoring and intervention. By taking into account comorbid conditions, the method can provide a more protective and preventative approach to care.

The cardiovascular health mapping function may be modified if the patient has a history of cardiovascular complications, resulting in a shortened cardiovascular health target interval. For example, patients with prior incidents of heart disease, high blood pressure, or other cardiovascular risks may have their intervals adjusted to reflect the need for more frequent follow-ups. This tailored approach to scheduling reflects the increased risk associated with cardiovascular issues, ensuring that follow-up care is provided at a frequency that is appropriate for patients with heightened cardiovascular concerns. By incorporating historical data into the mapping function, the method enhances the precision of care delivery and mitigates potential complications through earlier intervention.

The healthcare provider roles can be assigned based on the urgency levels associated with each health dimension score, with secondary roles escalated if the score remains alarming over consecutive appointments. For instance, a nurse may handle routine diabetes management for stable scores, while more critical cases may escalate to a specialist, such as a cardiologist or endocrinologist. By linking specific roles to urgency levels, the method facilitates efficient allocation of resources, ensuring that the most qualified healthcare providers are involved based on the patient's current needs. This dynamic allocation of roles improves responsiveness and ensures that high-risk cases are prioritized without overburdening specialist resources.

The appointment parameters may include consolidating follow-up care appointments for multiple health dimensions with close target dates, thereby enabling the method to recommend a single appointment. For example, if a patient's glucose control and cardiovascular health dimensions have target dates within a week of each other, the method may schedule one combined session to address both areas. Consolidation improves efficiency, reduces patient burden, and promotes coordinated care, as multiple health dimensions can be addressed in a single session when appropriate. This approach streamlines the patient experience while ensuring that no critical health dimensions are overlooked.

The method can be configured to identify secondary triggering health dimensions with target dates within a predetermined interval from the primary target date, allowing for a consolidated care approach where multiple dimensions are addressed in a single appointment. For example, secondary dimensions with target dates within two weeks of the primary date may be included in the appointment parameters. This feature optimizes the scheduling process by considering multiple health needs in parallel, streamlining care delivery. Incorporating secondary dimensions into the scheduling process reduces redundant appointments and ensures comprehensive care during each visit.

The diabetes resilience score can have an associated validity period, prompting the patient to retake the diabetes-specific questionnaire once the validity period has expired. For example, a high resilience score may remain valid for 18 months, while a low score may require reassessment after 3 months. This measure maintains the relevance of the resilience score by ensuring that it reflects the patient's most recent mental health status and diabetes distress, contributing to a more up-to-date assessment of their diabetes resilience. By using validity periods tailored to each patient's condition, the method ensures that follow-up intervals remain accurate and responsive to changes in patient well-being.

The method can be configured to generate reminders for patients and healthcare providers before the primary target date, and updates these reminders if the appointment is rescheduled. For instance, automated reminders may be sent via email, SMS, or app notifications one week before the scheduled date. Reminders improve adherence and prevent missed appointments, ensuring that both patient and provider are aligned on the care schedule. Dynamic updating of reminders accommodates any scheduling adjustments, maintaining communication and continuity in care. This feature enhances engagement and reduces the likelihood of missed follow-ups, ultimately improving treatment outcomes.

The method can be configured such that relevant lab tests are automatically ordered when cardiovascular health is identified as the triggering dimension, ensuring that lab results are available for the scheduled follow-up appointment. For example, blood tests for lipid profiles, HbA1c, or other relevant biomarkers may be ordered in advance. This proactive feature enhances the preparedness of the follow-up session, as the necessary diagnostic information is available for immediate review, allowing for a more effective consultation. By integrating diagnostic preparation into the scheduling process, the method minimizes delays in treatment and improves the quality of clinical decision-making.

In one embodiment, healthcare providers may override recommended scheduling and provider role assignments based on another fact, such as clinical judgment, allowing for personalized patient care. For example, a provider may choose to see a patient earlier than recommended if new symptoms arise, or assign a different specialist if unforeseen complications occur. This feature supports individualized treatment by enabling providers to adapt the automated recommendations based on specific patient needs and clinical insights. By balancing automation with provider discretion, the method ensures that care remains flexible and patient-centered, addressing unique medical scenarios as they arise.

The method can incorporates the use of patient preferences, such as preferred time, location, or healthcare provider, when scheduling follow-up appointments. Accommodating such preferences not only enhances patient engagement and satisfaction but also improves adherence to the care schedule. By aligning the care experience with the patient's specific needs and circumstances, the method fosters a patient-centered approach that supports long-term health management.

The method may involve a step of updating a summary of each appointment with details of health dimensions addressed and the last addressed date for each dimension covered. This summary provides a comprehensive and accessible record of past interactions, ensuring continuity of care and facilitating informed decision-making for future appointments. The updated last addressed dates are critical for tracking adherence to recommended care intervals and scheduling subsequent follow-ups.

Further, historical data for each health dimension can be tracked, allowing patients and healthcare providers to reference previously addressed health issues. This historical record serves as a valuable tool for identifying patterns and trends, enabling proactive adjustments to care strategies. By integrating this feature, the method enhances clinical insights and supports evidence-based decision-making, leading to improved outcomes over time.

The method may further be arranged such that a notification of the appointment parameters is provided to the patient and/or healthcare provider. This ensures that all relevant parties are informed of the appointment details, promoting communication and alignment in the care process.

The appointment parameters can for example be recorded into a device accessible by the patient, healthcare provider, and/or a central healthcare registry. By maintaining a shared record, the method facilitates collaborative care management, as all parties have access to up-to-date scheduling and care plans.

In a further aspect, the present disclosure relates to a system for managing diabetes care for a patient. The system may include a data acquisition module, a score-target interval mapping module, and an appointment management module, each of which may be configured to work collectively to enhance the efficiency and personalization of diabetes management.

The data acquisition module can be configured to collect diabetes health data. The data acquisition module may for example be configured to collect diabetes health data of the patient. The diabetes health data may comprise glucose monitoring data, electronic health records, patient-reported diabetes data, and fitness data.

The score-target interval mapping module may be configured to calculate a health dimension score, for example for each of a set of health dimensions derived from the collected diabetes health data. The diabetes health data may for example comprise or consist of glucose control, cardiovascular health, and diabetes resilience.

Alternatively or additionally, the score-target interval mapping module may be configured to map one or more, such as each, health dimension to a health target interval for a follow-up care appointment. The mapping may for example be based on each health dimension score and a mapping function specific to each health dimension. The health target intervals may comprise a glucose control target interval, a cardiovascular health target interval, and a diabetes resilience target interval.

The appointment management module may be configured to calculate target dates for each health dimension. This can for example be based on the elapsed time since the most recent appointment for that health dimension and its corresponding health target interval. The appointment management module may be configured to determine a triggering health dimension. The triggering health dimension may for example be the health dimension with the earliest target date. Alternatively, it could be the most urgent health dimension. The appointment management module may be configured to determine appointment parameters for the follow-up care appointment.

The appointment parameters may for example comprise a primary target date and/or a healthcare provider role. The primary target date for the follow-up care appointment can be determined based on the triggering health dimension. The healthcare provider role for the follow-up care appointment can be determined based on the triggering health dimension. Thus it can for example be the healthcare provider role associated with the triggering health dimension.

By integrating these components, the system may provide a dynamic and responsive method for managing diabetes care. The modular design may support scalability and customization, allowing healthcare providers to tailor the system to specific clinical protocols or patient demographics. Additionally, the inclusion of diverse data sources may offer a comprehensive and adaptive framework for addressing the varied and evolving needs of patients with diabetes.

The presently disclosed system may be configured to implement the method for managing diabetes care for a patient as described herein. Thus, the system may for example include modules and/or functionalities designed to perform the steps of the method. These may encompass collecting and processing diabetes health data, mapping health dimensions to target intervals, determining appointment parameters, and facilitating communication and coordination among patients and healthcare providers to ensure responsive and personalized care.

The data acquisition module can be designed to collect a variety of diabetes health data pertinent to the patient's condition. This module may gather glucose monitoring data, EHRs, patient-reported diabetes data, and fitness data, providing a comprehensive health profile that allows the system to make more informed decisions regarding follow-up care. By integrating data from multiple sources, the system ensures that it can monitor various aspects of the patient's health continuously, supporting real-time assessment and tailored diabetes management.

The score-target interval mapping module can be configured to calculates a health dimension score for each of the health dimensions derived from the diabetes health data. These dimensions include glucose control, cardiovascular health, and diabetes resilience. By evaluating these specific health dimensions, the system is better equipped to provide comprehensive diabetes management that addresses the multifaceted nature of the condition. This module not only calculates scores but also maps each health dimension to a corresponding target interval for follow-up care, allowing for a personalized interval-based care approach.

The score-target interval mapping module can be configured to map each health dimension to its respective health target interval using a mapping function tailored for each dimension, such as those specific to glucose control, cardiovascular health, and diabetes resilience. These health target intervals may vary depending on the urgency level associated with each dimension, thus enabling the system to prioritize follow-up appointments in a way that addresses each patient's specific health needs. A shorter interval for certain health dimensions may indicate a need for closer monitoring, while a longer interval may reflect stable control in a particular dimension.

The appointment management module can be configured to calculate target dates for each health dimension. These target dates are based on the elapsed time since the most recent appointment for the relevant health dimension and its associated health target interval. The module determines which health dimension has the earliest target date, thereby identifying a "triggering" health dimension, which serves as the basis for scheduling the next follow-up care appointment. This approach allows the system to allocate healthcare resources efficiently by identifying and prioritizing the dimension most in need of attention.

The appointment management module can be configured to determine the appointment parameters for the follow-up care appointment. These parameters include a primary target date, which is determined based on the triggering health dimension, and the healthcare provider role required for the appointment. By assigning a healthcare provider role aligned with the specific needs of the triggering health dimension, the system ensures that the patient receives the appropriate care from the most suitable healthcare provider. This approach supports efficient and targeted care, contributing to improved patient outcomes.

The data acquisition module may be configured to collect diabetes health data from diverse sources, such as EHRs, input devices like smartphones or tablets, CGM devices, and fitness tracking devices. By gathering data from such varied sources, the system can provide a holistic view of the patient's health, capturing both medical and lifestyle-related metrics essential for effective diabetes management.

The data acquisition module may include functionality for collecting patient-reported diabetes data from input devices, such as smartphones or tablets. This capability can enable patients to actively contribute data about their diabetes management, including subjective responses to questionnaires and self-recorded metrics. Such patient-reported data can enrich the system's data pool with insights that may not be captured through clinical measurements alone, improving the comprehensiveness of the system's analysis.

The data acquisition module may also be adapted to collect fitness data from a fitness tracking device. This data may include physical activity levels, sleep patterns, heart rate, and caloric expenditure. By incorporating fitness data, the system can align diabetes management with the patient's physical activities, offering more personalized care recommendations that promote overall health and wellness.

The data acquisition module may further be arranged to collect glucose monitoring data from either an input device or a CGM device. This flexibility can enable the system to utilize both patient-reported blood glucose readings and continuous glucose data from CGM devices, ensuring comprehensive glucose monitoring. Such adaptability may support real-time interventions by capturing glucose fluctuations and trends as they occur.

The system may include a data acquisition module capable of retrieving EHR data from a healthcare provider database or central health information system. This capability can allow the system to access laboratory test results, medical histories, prescribed medications, and other vital health records. With this in-depth clinical data, the system can calculate accurate health dimension scores and adjust care intervals effectively, ensuring tailored diabetes management.

Patient-reported diabetes data may comprise responses to diabetes-specific questionnaires, blood glucose entries, blood pressure measurements, and descriptive data such as weight and age. By integrating these patient-centric inputs, the system can gain valuable insights into individual factors that impact diabetes management, enabling a more tailored and precise approach to patient care.

EHRs accessed by the system may include laboratory test results, records of chronic conditions, demographic information, medical history, prescribed medications, and blood pressure readings. Such comprehensive medical data can equip the system to conduct a thorough analysis of the patient's health, facilitating accurate health dimension scoring and dynamic care adjustments.

The fitness data collected by the system may include physical activity metrics, sleep patterns, heart rate data, and caloric expenditure. By analyzing these lifestyle indicators, the system can make more nuanced care recommendations, accounting for the patient's daily habits and activity levels. This integrated approach may help promote better alignment between clinical care and the patient's overall health behaviors.

The glucose monitoring data may comprise CGM data and/or self-monitored blood glucose entries. Including both real-time CGM data and self-reported entries can provide a more complete picture of the patient's glucose patterns. This comprehensive data collection can enable the system to detect trends and fluctuations that inform timely and targeted diabetes care interventions.

The health dimension score for glucose control may be calculated based on glucose monitoring data collected over a predetermined time period. By analyzing glucose trends over time, the system can support early detection of glucose control issues, enabling proactive interventions that improve diabetes outcomes.

The glucose control score may be determined as a rolling average of glucose data over a specific period, such as a 30-day rolling average. This method can smooth out short-term fluctuations, providing a stable and reliable metric for assessing long-term glucose management. Such calculations may allow healthcare providers to make consistent and informed care adjustments.

The cardiovascular health score may be determined using a cardiovascular risk assessment method, such as SCORE2. By incorporating cardiovascular risk assessments, the system can ensure that patients with heightened cardiovascular risks are closely monitored, thereby mitigating potential complications associated with diabetes and cardiovascular health.

The cardiovascular risk assessment method may include factors such as blood pressure readings, lipid profile values, smoking status, and/or age. Including these specific indicators in the assessment provides a robust framework for evaluating cardiovascular health and making informed care recommendations. By integrating clinically relevant data, the system can prioritize patients at risk of cardiovascular complications and ensure timely follow-up care.

The diabetes resilience score may be derived from the patient's responses to a diabetes-specific questionnaire, such as the Problem Areas in Diabetes (PAID) questionnaire. This score can reflect the patient's psychological resilience and ability to cope with diabetes management challenges, supporting a more holistic approach to care. Using the PAID questionnaire allows the system to address the mental and emotional aspects of diabetes management, which are often overlooked in conventional care models. Other questionnaires are known to the skilled person.

The diabetes resilience score may also include an associated validity period. In this context, lower resilience scores can be assigned shorter validity periods, prompting the patient to retake the questionnaire as needed. This ensures that the score remains an accurate and current reflection of the patient's mental health status and coping capacity. The use of validity periods can also help identify changes in the patient's psychological resilience over time, allowing for timely interventions.

Each mapping function may be arranged to assign a health target interval based on the corresponding health dimension score. This approach can enable the system to adaptively schedule follow-up appointments based on real-time health scores, prioritizing care for dimensions with greater needs. For example, lower scores indicating poorer health can result in shorter target intervals, prompting more frequent follow-ups.

Each health dimension score may be mapped to its health target interval using a mapping function specific to each health dimension, such as glucose control, cardiovascular health, and diabetes resilience. By customizing the mapping functions, the system can tailor target intervals to reflect the specific care needs of each health dimension. For instance, the mapping function for cardiovascular health may include adjustments for patients with known cardiovascular complications.

Each mapping function may be defined as a piecewise function composed of at least three subfunctions. This piecewise approach allows the system to handle different score intervals flexibly, ensuring that each range of scores is mapped to a target interval that reflects the patient's needs. For example, scores in critical ranges may map to shorter target intervals, while stable scores may map to extended intervals.

The piecewise function's first interval may correspond to a critical score range, where follow-up care is urgently required. The second interval may represent moderate scores, where the mapping function provides a flexible and responsive adjustment to target intervals. The third interval may correspond to stable scores, where longer intervals are appropriate. This division ensures that follow-up frequency is dynamically aligned with the patient's condition.

The first interval may have a subfunction that sets a fixed health target interval, ensuring that patients in critical health states receive timely care. Similarly, the third interval may also use a constant subfunction to provide a stable follow-up schedule for patients with stable scores. These constant functions establish clear and predictable intervals for extreme health states, reducing variability and ensuring consistency in care.

In the second interval, the subfunction may have a non-zero derivative, introducing flexibility and enabling the target interval to adjust dynamically based on the patient's score within the intermediate range. This responsiveness is particularly valuable for patients transitioning between stable and critical health states, as it ensures care frequency adapts appropriately.

The subfunction in the second interval may be linear, offering a straightforward and proportional relationship between the health score and the target interval. This linearity simplifies the mapping process while maintaining precision in interval adjustments.

If one or more health dimension scores are unknown, the corresponding health target intervals may be assigned a predetermined default value. For instance, if glucose control data is unavailable due to device malfunction or patient non-compliance, the system can assign a conservative interval to ensure timely follow-up. This feature maintains continuity in care management by reducing the risk of missed or delayed appointments due to incomplete data.

The system may include functionality to adjust one or more health target intervals based on the presence of known complications, such as retinopathy, cardiovascular disease, or foot ulcers. Adjustments can involve shortening the interval for affected dimensions, ensuring that patients with severe or progressive conditions receive necessary care promptly. Such tailored intervals provide a preventative approach that mitigates the risk of complications.

The mapping function's thresholds for defining the intervals may be customizable, allowing healthcare providers to tailor the intervals based on clinical judgment or institutional guidelines. For instance, a clinic may lower the threshold for critical scores in populations with high cardiovascular risk, ensuring more proactive care.

One or more of the mapping functions may include a fourth subfunction corresponding to a fourth interval, which lies between the second and third intervals of the mapping function. The fourth interval may provide finer granularity for intermediate health scores, allowing for more precise adjustments to the health target interval. For example, this fourth subfunction can help identify cases where the patient's condition is transitioning between moderate and stable states, enabling timely and tailored follow-up care.

The fourth subfunction may be defined as a linear function. This linearity can ensure a straightforward and predictable adjustment to the health target interval within the fourth interval. For instance, a linear relationship may allow for incremental increases in follow-up intervals as the patient's condition improves within this transitional range. This approach simplifies the mapping while retaining flexibility for customized scheduling.

If one or more health dimension scores are unknown, the corresponding health target intervals may be assigned a predetermined default value. This ensures continuity in care management even when data gaps exist, such as during device malfunctions or periods of patient non-compliance. By assigning conservative default intervals, the system can minimize risks associated with missed or delayed care and ensure that patients still receive timely follow-up appointments.

The threshold values defining the intervals of each mapping function may be customizable based on clinic-specific criteria or healthcare provider preferences. For example, clinics may lower thresholds for high-risk populations to prioritize early intervention or adjust thresholds based on regional guidelines. Customization ensures alignment with local healthcare policies and provider expertise, enhancing the system's adaptability across various care settings.

One or more of the health mapping functions may be adjusted based on the presence of known complications, such as retinopathy, cardiovascular disease, or foot ulcers. Adjustments can involve shortening the health target intervals for affected dimensions, ensuring more frequent monitoring and timely interventions. For example, patients with severe complications may require significantly shorter intervals to reduce the risk of disease progression.

The cardiovascular health mapping function may be specifically adjusted for patients with a history of cardiovascular complications. This adjustment can result in shorter cardiovascular health target intervals, ensuring more frequent follow-ups for conditions such as hypertension, prior heart disease, or other cardiovascular risks. Such tailored adjustments can help mitigate risks by providing earlier detection and intervention for emerging cardiovascular issues.

Similarly, the glucose control mapping function may be adjusted for patients with diabetes-related complications, such as retinopathy or foot ulcers. Shortened glucose control target intervals can enable more frequent monitoring of blood glucose trends and earlier responses to deviations, reducing the likelihood of further complications. These tailored intervals enhance the precision of diabetes care for at-risk patients.

The diabetes resilience mapping function may also be adjusted for patients with known complications related to resilience, such as mental health challenges or high diabetes distress. A shortened diabetes resilience target interval can facilitate more frequent assessments of the patient's psychological coping capacity, ensuring timely support and interventions. By addressing these often-overlooked aspects of care, the system provides a more holistic approach to diabetes management.

The appointment management module may identify the triggering health dimension based on the primary target date and assign a healthcare provider role with primary responsibility for that dimension. This ensures that the most urgent health dimension is addressed by the appropriate provider, aligning care delivery with the patient's immediate needs. For example, if cardiovascular health is the triggering dimension, the module may assign a cardiologist or a general physician specializing in cardiovascular care.

Each health dimension may be associated with a healthcare provider role that has a primary responsibility for that dimension when the corresponding health dimension score is at a non-alarming level, such as within the first and/or second intervals. For instance, glucose control may be managed primarily by a nurse or diabetes educator for stable or moderately concerning scores. This allocation of primary roles ensures efficient use of healthcare resources while maintaining quality of care.

One or more health dimensions may also have an associated healthcare provider role with secondary responsibility. In such cases, the secondary role becomes active when the health dimension score is alarming, such as in the third interval. For example, a specialist, such as an endocrinologist or cardiologist, may take responsibility for health dimensions requiring escalated care. This dynamic allocation of roles supports efficient triaging and ensures that high-risk patients receive specialized attention when needed.

The healthcare provider roles can be structured to address specific health dimensions dynamically, ensuring that specialized care is aligned with the patient's needs. For example, a first role, a second role and a third role. In some examples the system comprises the use of a fourth role. In one example, the system may include distinct roles such as a doctor who can be assigned both primary and secondary responsibility for cardiovascular health, a nurse who may have primary responsibility for glucose control and diabetes resilience, and a mental health specialist who can take on secondary responsibility for diabetes resilience when the resilience score is alarming. This structured allocation may optimize resource utilization and can ensure that the appropriate expertise is applied based on the patient's health dimensions and urgency levels.

The system can further incorporate appointment parameters that account for secondary triggering health dimensions. These secondary dimensions may be identified when their target dates fall within a predetermined interval from the primary target date, allowing multiple aspects of the patient's health to be addressed during a single appointment. This coordination may reduce the need for separate visits and can promote a holistic approach to diabetes care, ensuring efficient and comprehensive management of the patient's condition.

In one example, if secondary triggering health dimensions are identified, the system may recommend consolidated or combined appointments through the appointment management module or an output module. This scheduling strategy can minimize disruptions for the patient and may enhance the efficiency of healthcare delivery by grouping related health concerns into a single visit. This feature may support a coordinated and patient-friendly care process.

The appointment management module can dynamically reschedule follow-up care appointments in response to real-time updates to health dimension scores or newly acquired health data. This capability may ensure that the scheduling of appointments remains flexible and responsive to the patient's evolving health conditions, enabling timely interventions that adapt to current needs. By integrating real-time data, the system may enhance the precision and effectiveness of diabetes care.

The system may also incorporate patient preferences, such as preferred time, location, or specific healthcare providers, into the appointment scheduling process. By considering these preferences, the system can support greater adherence and engagement from patients, as appointments are more likely to align with their personal circumstances. This customization may foster patient satisfaction and continuity in diabetes care.

Healthcare providers may override automated scheduling and/or role assignments through the appointment management module, allowing for adjustments based on clinical judgment. This flexibility can ensure personalized patient care, enabling providers to tailor appointments and roles to the unique requirements of each patient. By balancing automation with professional discretion, the system may enhance the adaptability of diabetes management.

The system, such as the appointment management module, or any other module, for example the output module, may generate automated reminders for patients and healthcare providers about upcoming target dates. Further, such reminders can be dynamically updated if appointments are rescheduled. These reminders can improve adherence to care schedules, ensuring that all parties are informed about upcoming appointments and any changes that occur. This functionality may reduce the risk of missed follow-ups and can strengthen the continuity of care.

The appointment summary module may allow healthcare providers to document the health dimensions addressed during each appointment and to update patient records accordingly. This feature can support continuity of care by enabling future providers to access comprehensive records of past interventions and decisions. Additionally, the module may automatically update the "last addressed date" for each dimension covered, ensuring adherence to recommended follow-up intervals.

The appointment summary module may also generate detailed summaries of each appointment, accessible to the patient, providing them with a clear record of their care. This transparency can encourage patient engagement by allowing individuals to review and understand the rationale behind their treatment plans. Such access may empower patients to take an active role in managing their diabetes, fostering a collaborative approach to care.

The system may include an appointment summary module configured to automatically update the "last addressed date" for each health dimension covered during an appointment. This functionality can ensure that an accurate record of addressed dimensions is maintained, supporting adherence to recommended care intervals and ensuring that follow-ups for each dimension occur in a timely manner. By updating the "last addressed date," the system may facilitate ongoing care planning based on the most current data.

The appointment summary module may be designed to generate a detailed summary of each appointment, which can be made accessible to the patient. This summary can provide a comprehensive record of the care received, allowing the patient to review the addressed health dimensions and the rationale behind each aspect of their treatment. This transparency may foster patient engagement, empowering individuals to actively participate in their diabetes management and facilitating better adherence to care plans.

The appointment summary module may further track and display historical data for each health dimension, enabling both patients and healthcare providers to reference previously addressed health issues. This capability can ensure continuity in diabetes management by highlighting patterns or recurring concerns in the patient's health history. The ability to access historical data may support informed decision-making for future appointments, enhancing the overall quality of care.

The system may include an output module configured to provide notifications of the appointment parameters. These notifications can ensure that patients and healthcare providers remain informed about upcoming appointments, promoting preparedness and reducing the likelihood of missed or delayed care. The notifications may include details such as the primary target date, healthcare provider role, and other relevant scheduling information.

The output module may send notifications directly to the patient and/or the healthcare provider assigned to the appointment. By targeting these communications to relevant parties, the system can enhance coordination and reduce the risk of scheduling conflicts or missed follow-ups. This feature may support effective communication and streamlined care processes.

The output module may also be configured to record the appointment parameters into a device accessible by the patient, the healthcare provider, and/or a central healthcare registry. This capability can ensure that a centralized and comprehensive record of scheduled appointments is maintained, supporting accurate tracking and enabling all authorized parties to access up-to-date scheduling information as needed.

The system may provide the appointment parameters as a recommendation to the healthcare provider through the output module. This recommendation can allow the provider to review, accept, or modify the suggested parameters before finalizing the appointment. This approach may balance automated scheduling with professional oversight, ensuring that the care plan aligns with both system recommendations and clinical judgment.

In a further aspect, the present disclosure relates to a computer program product stored on a computer-readable medium, comprising instructions which, when executed by a processor, cause the processor to perform the steps of the method as disclosed herein.

This product may be stored on a non-transitory computer-readable medium, such as a hard drive, flash memory, optical disc, or cloud-based storage. By executing these instructions, the computer program product supports various aspects of the diabetes care management system, including the collection of diabetes health data, calculation of health dimension scores, mapping of these scores to health target intervals, determination of appointment parameters, and the scheduling of follow-up care. This embodiment ensures that the entire method can be implemented in an automated, software-driven manner, providing flexibility and scalability for healthcare providers seeking to manage diabetes care for multiple patients effectively.

In yet a further aspect, the present disclosure relates to a non-transitory computer-readable medium comprising instructions that, when executed by a processor, cause the processor to perform the steps of the method as disclosed herein.

In a further aspect, the present disclosure relates to a device for managing diabetes care for a patient, comprising:
- a data acquisition module configured to collect diabetes health data of the patient, the diabetes health data comprising one or more of glucose monitoring data, electronic health records, patient-reported diabetes data, and fitness data;
- processing circuitry configured to:
   ∘ perform operations for calculating a health dimension score for each of a plurality of health dimensions derived from the collected diabetes health data, the health dimensions including glucose control, cardiovascular health, and diabetes resilience;
   ∘ perform operations for mapping each health dimension to a health target interval for a follow-up care appointment, wherein the mapping is based on each health dimension score and a mapping function specific to each health dimension, the health target intervals including a glucose control target interval, a cardiovascular health target interval, and a diabetes resilience target interval;
   ∘ perform operations for calculating target dates for each health dimension based on the elapsed time since the most recent appointment for that health dimension and its corresponding health target interval;
   ∘ perform operations for determining appointment parameters for the follow-up care appointment, the appointment parameters including:
      ▪ a primary target date for the follow-up care appointment, determined based on a triggering health dimension associated with the earliest target date among the plurality of health dimensions; and
      ▪ a healthcare provider role for the follow-up care appointment, determined based on the triggering health dimension.

The processing unit in the device may be configured to perform operations critical for managing diabetes care effectively. It can calculate a health dimension score for each of a plurality of health dimensions based on the collected diabetes health data. These health dimensions can include glucose control, cardiovascular health, and diabetes resilience, which provide a comprehensive assessment of the patient's condition across critical areas. By deriving these scores, the processing unit ensures that patient care is based on an integrated view of health metrics rather than isolated data points, enabling more informed decision-making.

The processing unit may further map each health dimension score to a corresponding health target interval for follow-up care appointments. This mapping may be performed using a dedicated mapping function specific to each health dimension, allowing the system to adaptively respond to the urgency associated with different health conditions. For instance, a lower health dimension score may correspond to a shorter follow-up interval, reflecting a higher priority for care, while a higher score may permit a longer interval. This feature enables the system to personalize follow-up schedules dynamically, improving patient outcomes by aligning care frequency with individual health needs.

In addition to mapping, the processing unit may calculate target dates for each health dimension based on the elapsed time since the most recent appointment for that dimension and its assigned health target interval. By continuously updating these target dates, the device can account for changes in the patient's health status and ensure that care remains timely and responsive. The processing unit may then identify the primary target date, which corresponds to the earliest target date among the health dimensions, designating the associated health dimension as the triggering health dimension. This prioritization mechanism allows the system to allocate resources efficiently, focusing on the most pressing aspect of the patient's health.

The processing unit may also assign a healthcare provider role suited to address the triggering health dimension. This assignment ensures that the patient is directed to a healthcare provider with the appropriate expertise, enhancing the relevance and effectiveness of the scheduled care. For example, the processing unit may recommend that a cardiologist be assigned to address cardiovascular health concerns or a mental health specialist for issues related to diabetes resilience. This dynamic assignment of provider roles further distinguishes the device by enabling a tailored and resource-efficient approach to diabetes management.

The device may for example be configured to carry out the method described herein. Thereby combining the functionality of a comprehensive diabetes care management system in a single device. This setup enables the device to autonomously collect data, process health scores, set target intervals, and schedule appointments, while adapting to real-time changes in the patient's health condition. By integrating all these capabilities into one device, this embodiment supports streamlined and effective diabetes management tailored to individual patient needs.

The device may further comprise additional components that can be useful for ensuring efficient operation and versatility. These components may include a power source, which can be a rechargeable battery, an external power connection, or other suitable energy supply systems to ensure uninterrupted functionality. The device may also include a non-transient computer-readable memory for storing diabetes health data, mapping functions, health dimension scores, calculated target dates, and patient history. Such a memory may further store program instructions executable by the processing circuitry to perform the operations described herein. Additionally, a communication interface may be provided, enabling the device to communicate with external systems, such as electronic health record (EHR) databases, patient monitoring devices, healthcare provider networks, or fitness tracking devices. This communication interface may include wired or wireless connections, such as Bluetooth, Wi-Fi, or cellular connectivity, to facilitate seamless data exchange.

The device may also comprise a display unit or a graphical user interface (GUI) configured to present calculated health dimension scores, health target intervals, or appointment parameters to patients and healthcare providers in an intuitive and accessible format. For example, the display unit may include a touchscreen or an integrated display for user interaction, allowing patients to input their diabetes-related information or review scheduled follow-up appointments. In some embodiments, an alerting module may be included to generate notifications, alarms, or reminders for upcoming appointments, thresholds exceeded in health dimension scores, or real-time adjustments to follow-up intervals. These alerts may be provided visually, audibly, or via notifications sent to connected devices, such as smartphones or tablets.

The device may further incorporate sensors or interfaces for collecting real-time diabetes health data, including continuous glucose monitoring (CGM) data, self-monitored blood glucose entries, and other patient health metrics. Such interfaces may enable direct integration with wearable health devices, fitness trackers, and remote monitoring tools, ensuring continuous data acquisition without manual input. Additionally, a data processing optimization module may be included to enhance the efficiency and accuracy of score calculations, mapping operations, and target date updates, ensuring that the device can perform complex operations with minimal latency.

In one implementation, the device may include a secure data encryption module to ensure that patient health data is protected during storage and transmission, meeting applicable data privacy regulations and standards such as GDPR or HIPAA. This security module may include encryption algorithms, secure access protocols, and authentication features to safeguard sensitive health information.

By integrating these components, the device is configured to autonomously perform the operations described, including collecting diabetes health data, calculating health dimension scores, mapping these scores to target intervals, determining triggering health dimensions, and scheduling follow-up care. These capabilities, combined with secure communication, storage, and display functionalities, make the device a comprehensive and adaptable solution for managing diabetes care. Such a setup supports personalized, real-time, and resource-efficient healthcare delivery, ensuring that patients receive care tailored to their individual needs and evolving health conditions.

### Detailed description of the drawings

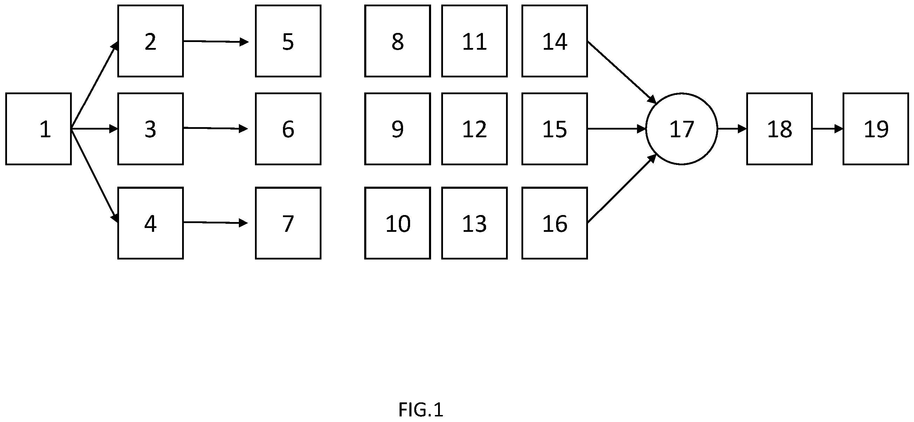

Figure 1 illustrates a flowchart of a computer-implemented method for managing diabetes care for a patient, according to an embodiment of the present disclosure. The method begins with collecting diabetes health data (1) of the patient. This health data may include glucose monitoring data, electronic health records, patient-reported diabetes data, and fitness data.

Using the collected diabetes health data (1), the system calculates a health dimension score for each of a set of health dimensions, including glucose control, cardiovascular health, and diabetes resilience. Specifically, the GLS health dimension score (2), the CVS health dimension score (3), and the DR health dimension score (4) are derived. For example, these scores may be represented as numerical values such as 65 for glucose control, 18 for cardiovascular health, and 86 for diabetes resilience.

Each health dimension score is then mapped to a health target interval for follow-up care. This is achieved by inputting the GLS health dimension score (2) into the GLS mapping function (5), the CVS health dimension score (3) into the CVS mapping function (6), and the DR health dimension score (4) into the DR mapping function (7). The results of these mapping functions yield the corresponding health target intervals: GLS health target interval (8), CVS health target interval (9), and DR health target interval (10). These target intervals reflect the urgency or priority associated with each health dimension.

Next, the elapsed time since the most recent appointment for each health dimension is factored in. Specifically, the system considers GLS elapsed time (11), CVS elapsed time (12), and DR elapsed time (13). Combining the respective elapsed times (11-13) with the health target intervals (8-10), the system calculates target dates for each health dimension. The resulting target dates include the GLS target date (14), the CVS target date (15), and the DR target date (16).

The system then determines the triggering health dimension (17), which is identified as the health dimension with the earliest target date among the diabetes-specific health dimensions. The triggering health dimension serves as the basis for prioritizing the upcoming follow-up care appointment.

Based on the triggering health dimension (17), the system determines the appointment parameters (18) for the follow-up care appointment. These parameters include a primary target date for the follow-up appointment, which is determined based on the target date of the triggering health dimension, and a healthcare provider role best suited to address the identified health dimension.

Finally, the system provides a notification (19) of the determined follow-up care appointment and its associated parameters. This notification may be sent to the patient and/or healthcare provider, ensuring timely communication and preparation for the scheduled appointment.

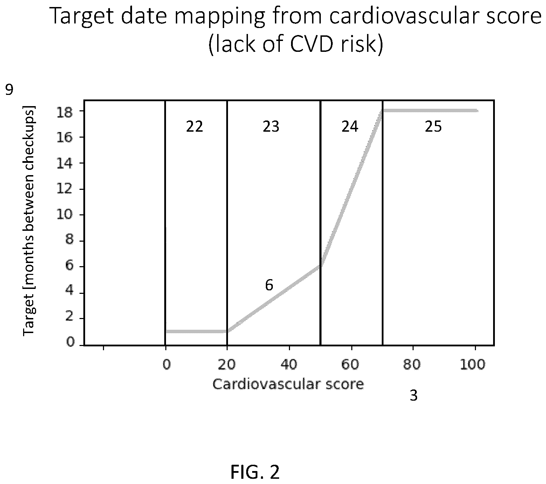

Figure 2 illustrates the cardiovascular health mapping function (6) that maps the cardiovascular health score (3) to a cardiovascular health target interval (9), measured in months between follow-up checkups, according to an embodiment of the present disclosure. The cardiovascular health score (3) is represented along the horizontal axis, while the corresponding health target interval (9) is depicted on the vertical axis. The mapping function (6) is defined as a piecewise function comprising a plurality of subfunction. In the example shown, there are four subfunctions over four intervals, each reflecting a different relationship between the cardiovascular health score (3) and the corresponding target interval (9).

In the first interval (22), corresponding to low cardiovascular health scores, the mapping function applies a first subfunction that sets a constant and short health target interval of approximately 1 month. This reflects a more urgent need for frequent monitoring and follow-up care in cases where the cardiovascular score (3) indicates significant cardiovascular health risk.

In the second interval (23), as the cardiovascular health score (3) increases, the mapping function applies a second subfunction with a linear increase in the health target interval (9). In this range, the target interval gradually extends as the cardiovascular score improves, signifying that lower cardiovascular risk warrants less frequent follow-ups. The linear increase provides a flexible and responsive adjustment to improving patient health.

The mapping function transitions to a fourth interval (24), which occurs between cardiovascular scores of approximately 50 and 70. In this interval, the mapping function applies a fourth subfunction with a steeper linear slope compared to the second subfunction. This means that the health target interval (9) increases more rapidly with improving cardiovascular scores in this range, reflecting a sharper transition towards extended follow-up intervals as the cardiovascular condition approaches a stable state.

In the third interval (25), which corresponds to cardiovascular scores above 70, the mapping function applies a third subfunction that sets a constant health target interval of approximately 18 months. This upper range reflects a state of stable cardiovascular health, where follow-up care can be less frequent without compromising the patient's condition.

If the cardiovascular health score (3) is unknown or unavailable, the system may assign a predetermined default value to the cardiovascular health target interval (9). For example, this predetermined default value may be set to 9 months, ensuring that follow-up care remains scheduled even when specific health data is missing.

The cardiovascular health mapping function (6) adapts dynamically across the four intervals, with each subfunction tailored to a specific cardiovascular score range. By applying constant and linear subfunctions as appropriate, the mapping ensures that follow-up care aligns with the urgency of the patient's cardiovascular health condition. Patients with lower cardiovascular scores receive frequent monitoring, while those with higher scores benefit from appropriately extended follow-up intervals, ensuring efficient and responsive care delivery.

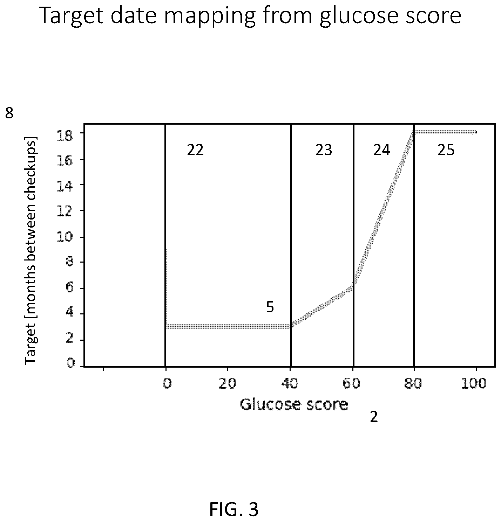

Figure 3 illustrates the glucose control mapping function (5) that maps the glucose control health dimension score (2) to a glucose control target interval (8) in months between follow-up checkups. Similar to the cardiovascular health mapping function shown in Figure 2, the glucose control mapping function (5) comprises four intervals, each defined by a corresponding subfunction.

In the first interval, for glucose control scores between 0 and 40, the target interval (8) is set to a constant value of 3 months, reflecting the need for frequent monitoring due to poor glucose control. The second interval spans scores between 40 and 60, where the target interval increases linearly as the glucose control score improves. Between scores of 60 and 80, the fourth interval applies a steeper linear increase, extending the target interval more rapidly as the glucose condition stabilizes. In the third interval, for glucose scores above 80, the target interval is set to a constant value of 18 months, indicating stable glucose control with less frequent follow-ups.

This mapping function (5) ensures dynamic adjustment of target intervals based on the glucose control score, mirroring the principles described for the cardiovascular mapping function in Figure 2. By applying constant and linear subfunctions across defined intervals, the system adapts follow-up care scheduling to reflect the patient's glucose control status, prioritizing frequent appointments for patients with poor glucose control and extending intervals as their condition improves.

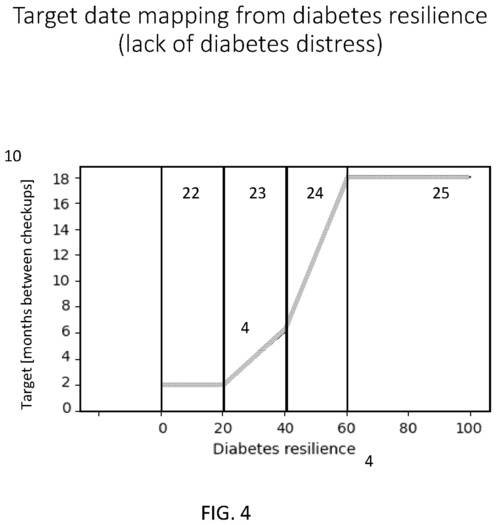

Figure 4 illustrates the diabetes resilience mapping function (7), which maps the diabetes resilience health dimension score (4) to a diabetes resilience target interval (10) in months between follow-up checkups. Similar to the mapping functions shown in Figures 2 and 3, the diabetes resilience mapping function comprises four intervals, each defined by a corresponding subfunction.

In the first interval, for diabetes resilience scores between 0 and 20, the target interval (10) is set to a constant value of 2 months, reflecting the need for close monitoring when resilience is low. The second interval, spanning scores between 20 and 40, shows a gradual linear increase in the target interval as the resilience score improves. For scores between 40 and 60, the fourth interval applies a steeper linear increase, further extending the target interval as resilience continues to stabilize. In the third interval, for scores above 60, the target interval remains constant at 18 months, indicating that patients with high diabetes resilience require less frequent follow-up.

This mapping function (7) dynamically adjusts target intervals based on the diabetes resilience score (4), following the same principles as the cardiovascular and glucose control mapping functions. The structure of constant and linearly increasing subfunctions ensures that patients with lower resilience scores are scheduled for closer monitoring, while those with higher scores are provided extended intervals, reflecting a stable mental and emotional condition.

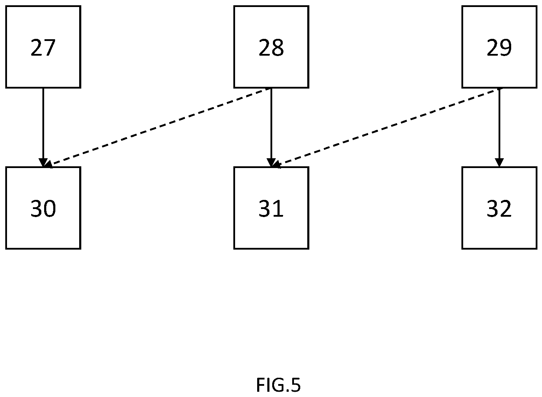

Figure 5 provides a schematic illustration of the dependency between the triggering health dimension and the corresponding healthcare provider role assigned for the follow-up appointment. The figure highlights the assignment logic for healthcare provider roles (30, 31, 32) based on the identified triggering health dimension (27, 28, 29) and distinguishes between non-alarming and alarming cases using solid and dashed lines, respectively.

The cardiovascular health dimension (27) is associated with a first healthcare provider role (30), which may represent a doctor or specialist responsible for cardiovascular care. This role is assigned both primary and secondary responsibility for cardiovascular health, ensuring that follow-up appointments are managed appropriately. In the case of alarming conditions, indicated by a score below a predetermined threshold, the role remains unchanged, as the first role is responsible for managing cardiovascular concerns across all urgency levels.

The glucose control health dimension (28) is typically assigned to the second healthcare provider role (31), which may represent a nurse or general healthcare provider. This role has primary responsibility for glucose control when the corresponding score is at a non-alarming level, such as within the first or second interval. However, in alarming cases, where the score falls below the predetermined threshold, the assignment is escalated to the first healthcare provider role (30) to ensure that higher clinical expertise is allocated to address the urgency of the condition.

The diabetes resilience health dimension (29) is normally assigned to the second healthcare provider role (31) when the resilience score is at a non-alarming level. However, when the diabetes resilience score indicates an alarming case, such as falling below a predetermined threshold, the assignment is made to the third healthcare provider role (32), which may represent a mental health specialist. This escalation ensures that patients experiencing significant challenges in diabetes resilience receive appropriate specialized care.

The figure further illustrates the flexibility of the system in dynamically assigning healthcare provider roles based on the health dimension score and its associated urgency level. The use of dashed lines represents alarming cases that trigger escalation to roles with secondary responsibility, while solid lines represent standard assignments under non-alarming conditions. This dependency structure supports efficient allocation of healthcare resources and ensures that patients receive care tailored to the priority and severity of their health needs.

### Items

1. A computer-implemented method for managing diabetes care for a patient, comprising:
   - collecting diabetes health data of the patient, including:
      ∘ glucose monitoring data,
      ∘ electronic health records,
      ∘ patient-reported diabetes data, and
      ∘ fitness data;
   - calculating a health dimension score for each of a set of health dimensions derived from the collected diabetes health data, wherein the health dimensions include glucose control, cardiovascular health, and diabetes resilience;
   - mapping each health dimension to a health target interval for a follow-up care appointment, based on each health dimension score and a mapping function specific to each health dimension, such as a glucose control mapping function, a cardiovascular health mapping function, and a diabetes resilience mapping function, the health target intervals comprising a glucose control target interval, a cardiovascular health target interval, and a diabetes resilience target interval;
   - calculating target dates for each health dimension based on the elapsed time since the most recent appointment for that health dimension and its corresponding health target interval;
   - determining a triggering health dimension, which is the health dimension with the earliest target date among the diabetes-specific health dimensions;
   - determining appointment parameters for the follow-up care appointment, wherein the appointment parameters include:
      ∘ a primary target date for the follow-up care appointment, determined based on the triggering health dimension, and
      ∘ a healthcare provider role for the follow-up care appointment, determined based on the triggering health dimension;
   - providing a notification of the determined follow-up care appointment and the appointment parameters.
2. The method according to item 1, wherein collecting diabetes health data comprises actively collecting measurement data from one or more patient monitoring devices, including a continuous glucose monitoring (CGM) device, an activity tracking device, or a self-monitoring blood glucose device, such as to obtain real-time data on the patient's health status.
3. The method according to any one of the preceding items, wherein the diabetes health data comprises data from electronic health records (EHRs), including laboratory test results, records of chronic conditions, demographic information, medical history, prescribed medications, and blood pressure readings.
4. The method according to any one of the preceding items, wherein the patient-reported diabetes data comprises responses to diabetes-specific questionnaires, blood glucose entries, blood pressure measurements, and/or descriptive data such as weight, and/or age.
5. The method according to any one of the preceding items, wherein the fitness data comprises physical activity metrics, sleep patterns, heart rate data, and/or caloric expenditure collected from a fitness tracking device.
6. The method according to any one of the preceding items, wherein calculating the health dimension scores includes applying weightings to the diabetes health data based on a clinical relevance to a condition of the patient.
7. The method according to any one of the preceding items, further comprising updating health dimension scores periodically to account for newly collected health data.
8. The method according to any one of the preceding items, wherein each health dimension score is mapped to a health target interval using a mapping function that assigns a variable target interval based on an urgency level, with the health target intervals comprising a glucose control target interval, a cardiovascular health target interval, and a diabetes resilience target interval.
9. The method according to any one of the preceding items, wherein the mapping function for each health dimension score is configured as a piecewise function with at least three intervals, each associated with a specific subfunction, including:
   - a first interval with a first subfunction,
   - a second interval with a second subfunction,
   - a third interval with a third subfunction.
10. The method according item 9, wherein the piecewise function's first and/or third subfunctions are constant functions, setting a fixed health target interval for each respective range, while the second subfunction has a non-zero derivative over the second interval.
11. The method according to any one of the preceding items, wherein, if one or more health dimension scores are unknown, the corresponding health target intervals are assigned a predetermined default value.
12. The method according to any one of the preceding items, wherein the health target intervals are adjusted based on the presence of known complications, such as retinopathy, cardiovascular disease, or foot ulcers, such that the health target interval of the corresponding health dimension is reduced.
13. The method according to any one of the preceding items, wherein the cardiovascular health mapping function is modified if the patient has a history of cardiovascular complications, resulting in a shortened cardiovascular health target interval.
14. The method according to any one of the preceding items, wherein the healthcare provider roles are determined based on urgency levels associated with each health dimension score, such that:
   - a healthcare provider role with primary responsibility is assigned for follow-up care appointment for non-urgent levels, such as when the health dimension score is within a good or moderate range (e.g., below a threshold level, such as within the first and/or second interval); and
   - a healthcare provider role with secondary responsibility is assigned for follow-up care appointment for urgent levels, such as when the health dimension score exceeds the threshold level (e.g., within the third interval).
15. The method according to any one of the preceding items, wherein each health dimension is associated with a healthcare provider role, such that:
   - the associated healthcare provider role with primary responsibility manages the health dimension when the health dimension score indicates a non-urgent level; and
   - the associated healthcare provider role with secondary responsibility manages the health dimension when the health dimension score indicates an urgent level.
16. The method according to any one of items 14-15, wherein healthcare provider roles are defined as follows:
   - a first role, such as a doctor, is assigned both primary and secondary responsibility for cardiovascular health;
   - a second role, such as a nurse, is assigned primary responsibility for glucose control and diabetes resilience; and
   - a third role, such as a mental health specialist, is assigned secondary responsibility for diabetes resilience when the resilience score indicates an urgent level.
17. The method according to any one of items 14-16, comprising dynamically reassigning healthcare provider roles if the health dimension score remains alarming over consecutive appointments, such that responsibility for the health dimension is escalated to a higher-level provider or specialist to address the escalating risk.
18. The method according to any one of the preceding items, wherein the appointment parameters include consolidating follow-up care appointments for multiple health dimensions with close target dates, allowing the system to recommend a single appointment.
19. The method according to any one of the preceding items, further comprising identifying one or more secondary triggering health dimensions that have target dates within a predetermined interval from the primary target date, and optionally wherein all triggering dimensions are included in the follow-up care appointment.
20. The method according to any one of the preceding items, wherein the health dimension score for glucose control is calculated based on glucose monitoring data collected over a predetermined time period.
21. The method according to any one of the preceding items, wherein the glucose control score is determined as a predetermined time rolling average of glucose monitoring data, such as the 30-day rolling average.
22. The method according to any one of the preceding items, wherein the cardiovascular health score is calculated using a cardiovascular risk assessment method, such as SCORE2.
23. The method according to item 22, wherein the cardiovascular risk assessment method incorporates blood pressure readings, lipid profile values, smoking status, and/or age.
24. The method according to any one of the preceding items, wherein the diabetes resilience score is based on results of the patient carrying out a diabetes-specific questionnaire, such as the Problem Areas in Diabetes (PAID) questionnaire.
25. The method according to any one of the preceding items, wherein the diabetes resilience score has an associated validity period, and the patient is prompted to retake the diabetes-specific questionnaire once the validity period has expired.
26. The method according to any one of the preceding items, further comprising generating reminders for patients and healthcare providers prior to the primary target date, and updating the reminder if the appointment is rescheduled.
27. The method according to any one of the preceding items, wherein relevant lab tests are automatically ordered when cardiovascular health is identified as the triggering dimension, to ensure lab results are available for the scheduled follow-up appointment.
28. The method according to any one of the preceding items, further comprising enabling healthcare providers to override recommended scheduling and provider role assignments based on clinical judgment, allowing for personalized patient care.
29. The method according to any one of the preceding items, wherein the system incorporates patient preferences such as preferred time, location, or healthcare provider when scheduling follow-up appointments to improve patient adherence and engagement.
30. The method according to any one of the preceding items, further comprising updating a summary of each appointment with details of health dimensions addressed and updating the last addressed date for each health dimension covered.
31. The method according to any one of the preceding items, further comprising tracking historical data for each health dimension, allowing both patients and healthcare providers to reference previously addressed health issues.
32. The method according to any one of the preceding items, wherein the notification is providing to the patient and/or a healthcare provider.
33. The method according to any one of the preceding items, further comprising recording the appointment parameters into a device accessible by the patient, the healthcare provider, and/or a central healthcare registry.
34. A system for managing diabetes care for a patient, comprising:
   - a data acquisition module configured to collect diabetes health data of the patient, including glucose monitoring data, electronic health records, patient-reported diabetes data, and fitness data;
   - a score-target interval mapping module configured to:
      ∘ calculate a health dimension score for each of a set of health dimensions derived from the collected diabetes health data, wherein the health dimensions include glucose control, cardiovascular health, and diabetes resilience; and
      ∘ map each health dimension to a health target interval for a follow-up care appointment, based on each health dimension score and a mapping function specific to each health dimension, such as a glucose control mapping function, a cardiovascular health mapping function, and a diabetes resilience mapping function, the health target intervals comprising a glucose control target interval, a cardiovascular health target interval, and a diabetes resilience target interval;
   - an appointment management module configured to:
      ∘ calculate target dates for each health dimension based on the elapsed time since the most recent appointment for that health dimension and its corresponding health target interval;
      ∘ determine a triggering health dimension which is the health dimension with the earliest target date;
      ∘ determine appointment parameters for the follow-up care appointment, wherein the appointment parameters includes:
         ∘ a primary target date for the follow-up care appointment, determined based on the triggering health dimension, among the diabetes-specific health dimensions; and
         ∘ a healthcare provider role for the follow-up care appointment, determined based on the triggering health dimension.
35. The system according to item 34, wherein the system is configured to carry out the method of any one of items 1-33.
36. The system according to any one of items 34-35, wherein the data acquisition module is adapted to collect the diabetes health data from electronic health records (EHRs), one or more input devices, a continuous glucose monitoring (CGM) device, and/or a fitness tracking device.
37. The system according to item 36, wherein the data acquisition module is adapted to collect the patient-reported diabetes data from the input device, such as a smartphone or a tablet.
38. The system according to any one of items 34-37, wherein the data acquisition module is adapted to collect the fitness data from the fitness tracking device.
39. The system according to any one of items 34-38, wherein the data acquisition module is adapted to collect the glucose monitoring data from the input device and/or the CGM device.
40. The system according to any one of items 34-39, wherein the data acquisition module is adapted to collect the electronic health records from a healthcare provider database or central health information system.
41. The system according to any one of items 34-40, wherein the patient-reported diabetes data comprises responses to diabetes-specific questionnaires, blood glucose entries, blood pressure measurements, and/or descriptive data such as weight and age.
42. The system according to any one of items 34-41, wherein the electronic health records (EHRs) comprise laboratory test results, records of chronic conditions, demographic information, medical history, prescribed medications, and blood pressure readings.
43. The system according to any one of items 34-42, wherein the fitness data comprises physical activity metrics, sleep patterns, heart rate data, and/or caloric expenditure.
44. The system according to any one of items 34-43, wherein the glucose monitoring data comprises continuous glucose monitoring (CGM) data and/or self-monitored blood glucose entries.
45. The system according to any one of items 34-44, wherein calculating the health dimension scores includes applying weightings to the diabetes health data based on clinical relevance to the patient's condition.
46. The system according to any one of items 34-45, wherein the health dimension score for glucose control is calculated based on glucose monitoring data collected over a predetermined time period.
47. The system according to item 46, wherein the glucose control score is determined as a predetermined time rolling average of glucose monitoring data, such as the 30-day rolling average.
48. The system according to any one of items 34-47, wherein the cardiovascular health score is calculated using a cardiovascular risk assessment method, such as SCORE2.
49. The system according to item 48, wherein the cardiovascular risk assessment method incorporates blood pressure readings, lipid profile values, smoking status, and/or age.
50. The system according to any one of items 34-49, wherein the diabetes resilience score is based on results of the patient carrying out a diabetes-specific questionnaire, such as the Problem Areas in Diabetes (PAID) questionnaire.
51. The system according to item 50, wherein the diabetes resilience score has an associated validity period, wherein lower resilience scores are assigned a shorter validity period than higher resilience scores, and wherein the system prompts the patient to retake the diabetes-specific questionnaire once the validity period has expired.
52. The system according to any one of items 34-51, wherein each mapping function assigns the corresponding health target interval based on the corresponding health dimension score.
53. The system according to any one of items 34-52, wherein each health dimension score is mapped to the corresponding health target interval using a dedicated mapping function specific to each health dimension.
54. The system according to any one of items 34-53, wherein each mapping function is defined as a piecewise function comprising at least three subfunctions.
55. The system according to item 54, wherein the at least three subfunctions collectively form the mapping function over at least three intervals of the corresponding health dimension score, including:
   - a first interval, over which the mapping function is a first subfunction;
   - a second interval, over which the mapping function is a second subfunction;
   - a third interval, over which the mapping function is a third subfunction.
56. The system according to item 55, wherein the first interval is lower than the second interval, which is lower than the third interval.
57. The system according to item 55-56, wherein the first subfunction and/or the third subfunction are constant functions, setting a fixed health target interval.
58. The system according to any one of items 55-57, wherein the second subfunction has a non-zero derivative over the entire second interval.
59. The system according to any one of items 55-58, wherein the second subfunction is a linear function.
60. The system according to any one of items 34-59, wherein one or more of the mapping functions has a fourth subfunction over a fourth interval, wherein the fourth interval is between the second and third interval, and wherein the fourth subfunction has a non-zero derivative.
61. The system according to item 60, wherein the fourth subfunction is a linear function.
62. The system according to any one of items 34-61, wherein, if one or more of the health dimension scores are unknown, the corresponding health target intervals are assigned a predetermined default value.
63. The system according to any one of items 34-62, wherein the threshold values defining the intervals of each mapping function are customizable based on clinic-specific criteria or healthcare provider preferences.
64. The system according to any one of items 34-63, wherein one or more of the health mapping functions is adjusted based on the presence of known complications, such as retinopathy, cardiovascular disease, or foot ulcers, to reduce the health target interval of the corresponding health dimension.
65. The system according to item 64, wherein the cardiovascular health mapping function is adjusted if the patient has a history of cardiovascular complications, resulting in a shortened cardiovascular health target interval for cardiovascular health follow-up appointments.
66. The system according to any one of items 34-65, wherein the glucose control mapping function is adjusted if the patient has a history of diabetes-related complications, such as retinopathy or foot ulcers, resulting in a shortened glucose control target interval for glucose control follow-up appointments.
67. The system according to any one of items 34-66, wherein the diabetes resilience mapping function is adjusted if the patient has a history of diabetes resilience complications, resulting in a shortened diabetes resilience target interval for diabetes resilience follow-up appointments.
68. The system according to any one of items 34-67, wherein the appointment management module identifies the triggering health dimension based on the primary target date and assigns a healthcare provider role with primary responsibility for that dimension.
69. The system according to item 68, wherein each health dimension is associated with a healthcare provider role that has a primary responsibility, such that the associated healthcare provider role has the responsibility of this health dimension when the associated health dimension score is at a non-urgent level, such as a good level, and/or a moderate level, for example below a threshold level, for example within the first and/or the second interval.
70. The system according to item 69, wherein one or more of the health dimensions have an associated healthcare provider role with a secondary responsibility, such that the associated healthcare provider role has the responsibility of this health dimension when the associated health dimension score is at an urgent level, such as above a threshold level, for example when the health dimension is in the third interval.
71. The system according to item 70, wherein the healthcare provider roles comprise:
   - a first role, such as a doctor, assigned both primary and secondary responsibility for cardiovascular health;
   - a second role, such as a nurse, assigned primary responsibility for glucose control and diabetes resilience;
   - a third role, such as a mental health specialist, assigned secondary responsibility for diabetes resilience when the resilience score is at an urgent level.
72. The system according to any one of items 34-62, wherein the appointment parameters include the identification and determination of one or more secondary triggering health dimensions, which are health dimensions with a target date that is within a predetermined interval from the primary target date.
73. The system according to item 72, wherein the appointment management module or the output module recommends a consolidated or combined appointment if secondary triggering health dimensions are identified.
74. The system according to any one of items 34-73, wherein the appointment management module dynamically reschedules follow-up care appointments in response to real-time updates to health dimension scores or newly acquired health data.
75. The system according to any one of items 34-74, wherein the appointment management module incorporates patient preferences, such as preferred time, location, or healthcare provider, when scheduling follow-up appointments to improve patient adherence and engagement.
76. The system according to any one of items 34-75, wherein the appointment management module enables healthcare providers to override recommended scheduling and provider role assignments based on clinical judgment, allowing for personalized patient care.
77. The system according to any one of items 34-76, wherein the appointment management module provides automated reminders to patients and healthcare providers based on upcoming target dates and updates these reminders if appointments are rescheduled.
78. The system according to any one of items 34-77, comprising an output module configured to provide a notification of the appointment parameters.
79. The system according to item 78, wherein the output module sends notifications to the patient and/or the healthcare provider assigned to the appointment.
80. The system according to any one of items 78-79, wherein the output module is configured to schedule the follow-up appointment by recording the appointment parameters into a device accessible by the patient, the healthcare provider, and/or a central healthcare registry.
81. The system according to any one of items 34-80, wherein the output module provides the appointment parameters as a recommendation to the healthcare provider, enabling acceptance or modification before finalizing the follow-up appointment.
82. The system according to any one of items 34-81, further comprising an appointment summary module configured to allow healthcare providers to document the health dimensions addressed during each appointment and to update patient records accordingly.
83. The system according to item 82, wherein the appointment summary module automatically updates the "last addressed date" for each health dimension covered during an appointment, supporting historical tracking and adherence to interval recommendations.
84. The system according to any one of items 82-83, wherein the appointment summary module generates a detailed summary of each appointment for patient access, providing a comprehensive record of care.
85. The system according to any one of items 82-84, wherein the appointment summary module tracks and displays historical data for each health dimension, allowing both patients and healthcare providers to reference previously addressed health issues.
86. The system according to any one of items 34-85, comprising an output module configured to provide a notification of the appointment parameters.
87. The system according to item 86, wherein the output module sends notifications to the patient and/or the healthcare provider assigned to the appointment.
88. The system according to any one of items 86-87, wherein the output module is configured to schedule the follow-up appointment by recording the appointment parameters into a device accessible by the patient, the healthcare provider, and/or a central healthcare registry.
89. The system according to any one of items 86-88, wherein the output module provides the appointment parameters as a recommendation to the healthcare provider, enabling acceptance or modification before finalizing the follow-up appointment.
90. A computer program product comprising instructions which, when executed by a processor, cause a computer to carry out the method according to any one of items 1-33.
91. A device for managing diabetes care for a patient, comprising:
   - a data acquisition module configured to collect diabetes health data of the patient, including glucose monitoring data, electronic health records, patient-reported diabetes data, and fitness data;
   - a processing unit configured to:
      ∘ calculate a health dimension score for each of a set of health dimensions derived from the collected diabetes health data, wherein the health dimensions include glucose control, cardiovascular health, and diabetes resilience;
      ∘ map each health dimension to a health target interval for a follow-up care appointment, based on each health dimension score and a mapping function specific to each health dimension, the health target intervals comprising a glucose control target interval, a cardiovascular health target interval, and a diabetes resilience target interval;
      ∘ calculate target dates for each health dimension based on the elapsed time since the most recent appointment for that health dimension and its corresponding health target interval;
      ∘ determine appointment parameters for the follow-up care appointment, including a primary target date for the follow-up care appointment, determined based on a triggering health dimension which has the earliest target date among the diabetes-specific health dimensions, and a healthcare provider role for the follow-up care appointment, determined based on the triggering health dimension.
92. The device according to item 91, wherein the device is arranged to carry out the method of any one of items 1-33.

## Claims

1. A computer-implemented method for managing diabetes care for a patient, comprising:
• collecting diabetes health data of the patient, including:
∘ glucose monitoring data,
∘ electronic health records,
∘ patient-reported diabetes data, and
∘ fitness data;
• calculating a health dimension score for each of a set of health dimensions derived from the collected diabetes health data, wherein the health dimensions include glucose control, cardiovascular health, and diabetes resilience, the health dimension score reflecting an urgency level;
• mapping each health dimension to a health target interval for a follow-up care appointment, based on each health dimension score and a mapping function specific to each health dimension, such as a glucose control mapping function, a cardiovascular health mapping function, and a diabetes resilience mapping function, the health target intervals comprising a glucose control target interval, a cardiovascular health target interval, and a diabetes resilience target interval;
• calculating target dates for each health dimension based on the elapsed time since the most recent appointment for that health dimension and its corresponding health target interval;
• determining a triggering health dimension, which is the health dimension with the earliest target date among the diabetes-specific health dimensions;
• determining appointment parameters for the follow-up care appointment, wherein the appointment parameters include:
∘ a primary target date for the follow-up care appointment, determined based on the triggering health dimension, and
∘ a healthcare provider role for the follow-up care appointment, determined based on the triggering health dimension;
• providing a notification of the determined follow-up care appointment and the appointment parameters.

2. The method according to claim 1, wherein collecting diabetes health data comprises actively collecting measurement data from one or more patient monitoring devices, including a continuous glucose monitoring (CGM) device, an activity tracking device, or a self-monitoring blood glucose device, such as to obtain real-time data on the patient's health status.

3. The method according to any one of the preceding claims, wherein the diabetes health data comprises data from electronic health records (EHRs), including laboratory test results, records of chronic conditions, demographic information, medical history, prescribed medications, and blood pressure readings.

4. The method according to any one of the preceding claims, wherein the patient-reported diabetes data comprises responses to diabetes-specific questionnaires, blood glucose entries, blood pressure measurements, and/or descriptive data such as weight, and/or age.

5. The method according to any one of the preceding claims, wherein the fitness data comprises physical activity metrics, sleep patterns, heart rate data, and/or caloric expenditure collected from a fitness tracking device.

6. The method according to any one of the preceding claims, wherein calculating the health dimension scores includes applying weightings to the diabetes health data based on predefined clinical guidelines or dynamically adjusted factors reflecting the patient's health condition.

7. The method according to any one of the preceding claims, further comprising dynamically or periodically updating the health dimension scores in response to newly collected diabetes health data, wherein the updates reflect changes in the patient's condition over time, and the collected data comprises inputs from continuous monitoring devices, electronic health records, patient-reported data, and/or fitness tracking devices..

8. The method according to any one of the preceding claims, wherein each health dimension score is mapped to a health target interval using a mapping function that assigns a variable target interval based on the urgency level, with the health target intervals comprising a glucose control target interval, a cardiovascular health target interval, and a diabetes resilience target interval.

9. The method according to any one of the preceding claims, wherein the mapping function specific to each health dimension comprises a piecewise function with at least three intervals, each associated with a specific subfunction, including:
• a first interval with a first subfunction,
• a second interval with a second subfunction,
• a third interval with a third subfunction.

10. The method according claim 9, wherein the first and/or third subfunctions of each mapping function are constant functions, setting fixed health target intervals for the corresponding ranges, and the second subfunction is defined by a non-zero derivative over its respective interval..

11. The method according to any one of the preceding claims, wherein the health target intervals are adjusted based on the presence of known complications, such as retinopathy, cardiovascular disease, or foot ulcers, such that the health target interval of the corresponding health dimension is reduced.

12. The method according to any one of the preceding claims, wherein the healthcare provider roles are determined based on the urgency levels associated with each health dimension score, such that:
• a healthcare provider role with primary responsibility is assigned for follow-up care appointment for non-urgent levels, such as when the health dimension score is within a good or moderate range (e.g., below a threshold level, such as within the first and/or second interval); and
• a healthcare provider role with secondary responsibility is assigned for follow-up care appointment for urgent levels, such as when the health dimension score exceeds the threshold level (e.g., within the third interval).

13. The method according to claim 12, comprising dynamically reassigning the healthcare provider role associated with one of the health dimensions to the healthcare provider role with secondary responsibility for the health dimension, if the health dimension score remains at an urgent level over consecutive follow-up appointments.

14. The method according to any one of the preceding claims, wherein the health dimension score for glucose control is calculated based on glucose monitoring data collected over a predetermined time period.

15. A system for managing diabetes care for a patient, comprising:
• a data acquisition module configured to collect diabetes health data of the patient, including glucose monitoring data, electronic health records, patient-reported diabetes data, and fitness data;
• a score-target interval mapping module configured to:
∘ calculate a health dimension score for each of a set of health dimensions derived from the collected diabetes health data, wherein the health dimensions include glucose control, cardiovascular health, and diabetes resilience; and
∘ map each health dimension to a health target interval for a follow-up care appointment, based on each health dimension score and a mapping function specific to each health dimension, such as a glucose control mapping function, a cardiovascular health mapping function, and a diabetes resilience mapping function, the health target intervals comprising a glucose control target interval, a cardiovascular health target interval, and a diabetes resilience target interval;
• an appointment management module configured to:
∘ calculate target dates for each health dimension based on the elapsed time since the most recent appointment for that health dimension and its corresponding health target interval;
∘ determine a triggering health dimension which is the health dimension with the earliest target date;
∘ determine appointment parameters for the follow-up care appointment, wherein the appointment parameters includes:
∘ a primary target date for the follow-up care appointment, determined based on the triggering health dimension, among the diabetes-specific health dimensions; and
∘ a healthcare provider role for the follow-up care appointment, determined based on the triggering health dimension.
